# EUROPEAN PATENT SPECIFICATION

(11) **EP 0 377 906 B1**
(45) Date of publication and mention of the grant of the patent: **15.12.1993**
(21) Application number: 89124167.1
(22) Date of filing: 30.12.1989
(51) Int. Cl.: A61K 9/46, A61K 33/10

(54) **Effervescent analgesic antacid composition having reduced sodium content**
Analgetische antacide Brausezusammensetzung mit reduziertem Natriumgehalt
Composition effervescente antacide analgésique à teneur de sodium réduite

(30) Priority: 12.01.1989 US 296537; 09.05.1989 US 349113; 05.09.1989 US 401064
(43) Date of publication of application: 18.07.1990
(73) Proprietor: BAYER AG, 51368 Leverkusen (DE)
(72) Inventor: Duvall, Nash Ronald, Elkhart, IN 46514 (US); Gold, Gerald, Elkhart, IN 46514 (US)

(56) References cited:
- EP-A- 0 203 768
- DE-A- 1 800 727
- US-A- 3 401 216
- US-A- 4 650 669
- US-A- 4 678 661
- US-A- 4 704 269

## Description

### BACKGROUND OF THE INVENTION AND PRIOR ART

Effervescent analgesic antacid compositions containing acetylsalicylic acid as the analgesic component and citric acid and sodium bicarbonate as the principal ingredients of an effervescent couple have been known for many years. One of the disadvantages of these compositions is the elevated sodium content which renders them unsuitable for individuals who should reduce their sodium intake. While efforts have been made in the prior art to produce such compositions having reduced sodium content by including calcium carbonate and potassium bicarbonate, for example, the resulting products form solutions that have an unpleasant taste. When acetaminophen, which has an unpleasant taste itself, is used to replace all or a part of the acetylsalicylic acid as the analgesic component, the resulting product has been generally unacceptable from a taste standpoint.

Another problem with prior art effervescent analgesic antacid compositions having reduced sodium content is that they do not completely dissolve. They form a cloudy or milky solution with a scum of undissolved particles floating on the surface of the liquid.

Ketoprofen is another analgesic compound that is suitable for use in an effervescent analgesic antacid composition.

There is thus a need for an effervescent analgesic antacid composition containing acetylsalicylic acid, acetaminophen, ketoprofen or mixtures thereof as the analgesic component and reduced sodium content in the effervescent couple/antacid component which forms a solution that is pleasant tasting. There is also a need for such composition that will substantially completely dissolve in water to form a clear solution with no scum on the liquid surface.

U.S. Patent No. 3,495,001 discloses a sodium-free effervescent analgesic composition. U.S. Patent Nos. 2,854,377; 2,953,459; 2,985,562; 3,102,075; 3,105,792; 3,136,692; 3,243,377; 3,518,343; 3,903,255; and 4,093,710 disclose various effervescent compositions containing various amounts and combinations of glycine, surfactants such as dioctyl sodium sulfosuccinate, fumaric acid and polyvinyl pyrrolidone. I.R.Mohrle, "Pharmaceutical Dosage Forms: Tablets", Vol. 1, Marcel Dekker, Inc., New York, NY, pp. 225-258 (1980) provides a full description of various effervescent tablet formulations and their ingredients. U.S. Patent No. 4,704,269 discloses an effervescent analgesic antacid composition having reduced sodium content wherein the antacid and a food grade acid reactive therewith to form the effervescent couple are in the form of an agglomerate held together by a water soluble food grade binder.

EP-A-0 203 768 discloses a composition containing calcium carbonate which dissolves in cold water in about 1 minute. This quick solubility is achieved by employing specific small particle sizes for the therapeutic agent and one of the components of the effervescent system. US-A-4 704 269 only discloses use of potassium bicarbonate, either alone or in mixture with sodium antacid material. There is no disclosure or suggestion of the inclusion of calcium carbonate in the effervescent couple, even though the overall amounts of the antacid mixture are similar to the present compositions.

None of the above prior art disclosures specifically disclose or suggest the novel compositions of the present invention.

### SUMMARY OF THE INVENTION

According to this invention, there is provided an effervescent analgesic antacid composition having a reduced sodium content which is capable of being substantially completely dissolved in water to form a pleasant tasting solution which comprises a mixture of 0.2 - 16 % of an analgesic selected from the class consisting of acetylsalicylic acid, acetaminophen, ketoprofen and mixtures thereof, 26 - 40 % citric acid, 0 - 14 % glycine, 0.8 - 1.5 % flavors and sweeteners, 0 - 31 % tableting aids other than lubricants and 0 - 6 % tablet lubricant other than acetylsalicylic acid and an antacid component characterized by containing 13 - 21 % sodium bicarbonate, 7 - 12 % calcium carbonate and 8 - 14 % potassium bicarbonate, said percents being weight percent based on the total weight of the composition.

### DESCRIPTION OF THE INVENTION

Acetylsalicylic acid, acetaminophen, ketoprofen or a mixture thereof provides the analgesic component of this composition. The antacid component is provided primarily by a mixture of sodium bicarbonate, calcium carbonate, and potassium bicarbonate. The effervescent couple is provided by citric acid reacting with the carbonates and bicarbonates of the antacid component.

When acetylsalicylic acid, acetaminophen or mixture thereof is the analgesic, it is employed in an amount to produce a dose containing 325-500 mg. of the analgesic. When ketoprofen is the analgesic, it is employed in an amount to produce a dose containing 6.25-50 mg. of the analgesic. The calcium carbonate should be employed in an amount so as to provide a total daily dosage not exceeding 8 g. The calcium carbonate is preferably employed in the spray-dried form described in U.S. Patent No. 4,650,669. The potassium bicarbonate is employed in an amount not to exceed a total daily dose of 2.5 g. If desired, glycine may be employed to achieve a desired level of acid neutralizing capacity. The resulting composition when dissolved in water produces a pH of 4-6.

The taste of the product after it is dissolved in water can be improved by including in the composition minor amounts of flavors, such as lemon, grapefruit and orange flavors, as well as sweeteners, such as aspartame and calcium or sodium saccharin. The aspartame may be used in the form of granules containing lactose and a nonionic surfactant as described in U.S. Patent No. 4,783,331.

This composition can be used in a powder-granulated form or it can be used in the form of compressed tablets. In the production of tablets a lubricant is necessary for the tablet dies. When a significant amount of acetylsalicylic acid is present in the formulation, it will function as a lubricant. When acetylsalicylic acid is not used or is present in minor amounts, it is desirable for fumaric acid to be used as a lubricant. It is understood, however, that other well-known tablet lubricants, such as adipic acid and sodium benzoate, can also be used. It is also preferable to include tableting aids other than lubricants, such as inert fillers or binders. Examples of such fillers or binders are sorbitol, lactose, mannitol, fructose, sucrose, a co-crystallized mixture of 97% sucrose and 3% modified dextrins or hydroxypropylmethylcellulose. It is preferred that the major component of the tableting aids other than lubricants be sorbitol.

In order to have a substantially completely dissolved product with no scum floating on the liquid surface, it is preferable to include in the composition minor amounts of polyvinyl pyrrolidone, organopolysiloxane (such as dimethyl polysiloxane), and dioctyl sodium sulfosuccinate surfactant.

The composition of the present invention contains 0.2-16% of an analgesic selected from the class consisting of acetylsalicylic acid, acetaminophen, ketoprofen and mixtures thereof, 26-40% citric acid, 13-21% sodium bicarbonate, 7-12% calcium carbonate, 8-14% potassium bicarbonate, 0-14% glycine, 0.8-1.5% flavors and sweeteners, 0-31% tableting aids other than lubricants, and 0-6% tablet lubricant other than acetylsalicylic acid. Preferably, the composition contains 0.2-16% acetylsalicylic acid, acetaminophen, ketoprofen or mixtures thereof, 26-30% citric acid, 13-16% sodium bicarbonate, 7-9% calcium carbonate, 8-10% potassium bicarbonate, 0-13% glycine, 0.8-1.5% flavors and sweeteners, 11-26% tableting aids other than lubricants, 2-6% fumaric acid, 0.03-0.04% polyvinyl pyrrolidone, 0.01-0.02% organopolysiloxane, and 0.001-0.002% dioctyl sodium sulfosuccinate. All of the above percents are weight percent based on the total weight of the composition.

The final form of the composition is produced by dry blending all the ingredients. Final tablet forms are produced by feeding the above mixture to a tablet press in a manner known to those skilled in the art.

The following example describes production of tablets of one form of the preferred composition.

Due to accuracy of calculation the sum of the components does not total exactly 100%

### EXAMPLE 1

A 5100 g. quantity of granulated acetaminophen (containing 95.6 weight percent acetaminophen, 3.8 weight percent citric acid, and 0.6 weight percent hydroxypropylmethylcellulose) was passed through a Fitzpatrick Comminutor Model D operating at 4500 rpm. A 6000 g. quantity of glycine and a 4500 g. quantity of potassium bicarbonate were separately dried at 130° F. (54.44° C.) for 16 hr. A mixture of 5955.9 g. sorbitol, 15 g. polyvinyl pyrrolidone, 7.5 g. of dimethyl polysiloxane, 0.75 g. dioctyl sodium sulfosuccinate (in the form of a mixture containing 85% dioctyl sodium sulfosuccinate and 15% sodium benzoate), 4500 g. above-dried potassium bicarbonate and 1425 g. fumaric acid was rough-mixed by passing it through a Fitzpatrick Comminutor Model D at 2500 rpm. This latter mixture was then final mixed with the above acetaminophen and glycine along with 250.5 g. of a mixture of lemon, grapefruit and orange flavors, 550.35 g. of aspartame granules (of the type described in U.S. Patent No. 4,783,331 containing 78.61% lactose, 0.95% nonionic surfactant and 20.44% aspartame), 45 g. calcium saccharin, 4500 g. spray-dried calcium carbonate (containing 83% calcium carbonate, 9.95% lactose and 7.05% maltodextrin of the type described in U.S. Patent No. 4,650,669), 13275 g. anhydrous citric acid and 7125 g. sodium bicarbonate that had been heat-treated as described in U.S. Patent No. 3,105,792 in a 3 cu. ft. V-Blender for 15 minutes. The final mixture was then fed to a tablet press to produce tablets each containing 325 mg. acetaminophen and having a composition of:

| Weight % | Ingredient |
|---|---|
| 10.00 | Acetaminophen |
| 27.63 | Citric Acid |
| 14.62 | Sodium Bicarbonate |
| 7.66 | Calcium Carbonate |
| 9.23 | Potassium Bicarbonate |
| 12.31 | Glycine |
| 0.84 | Flavors and Sweeteners |
| 14.75 | Sorbitol and Other Tableting Aids Other than Lubricants |
| 2.92 | Fumaric Acid |
| 0.03 | Polyvinyl Pyrrolidone |
| 0.02 | Dimethyl Polysiloxane |
| 0.002 | Dioctyl Sodium Sulfosuccinate |
| 1̅0̅0̅.̅0̅1̅2̅ | |

When one or two of the above-produced tablets were placed in a glass containing about 4 oz. (118 ml.) water, there was significant effervescence while the tablet(s) dissolved resulting in a substantially clear solution with no scum on the liquid surface. This solution had a pleasant taste with no undesirable after-taste.

The following examples describe production of other forms of the composition of this invention.

### EXAMPLE 2

The formulation of Example 1 is modified to increase the tablet content of acetaminophen to 500 mg. The sorbitol content is reduced to compensate for this keeping all the other ingredients the same. The tablet product has the composition of:

| Weight % | Ingredient |
|---|---|
| 15.00 | Acetaminophen |
| 27.15 | Citric Acid |
| 14.25 | Sodium Bicarbonate |
| 7.47 | Calcium Carbonate |
| 9.00 | Potassium Bicarbonate |
| 12.00 | Glycine |
| 0.82 | Flavors and Sweeteners |
| 11.41 | Tableting Aids Other than Lubricants |
| 2.85 | Fumaric Acid |
| 0.03 | Polyvinyl Pyrrolidone |
| 0.02 | Dimethyl Polysiloxane |
| 0.002 | Dioctyl Sodium Sulfosuccinate |
| 1̅0̅0̅.̅0̅0̅2̅ | |

### EXAMPLE 3

The formulation of Example 1 is used with the direct substitution of acetylsalicylic acid for acetaminophen. The fumaric acid is deleted since the acetylsalicylic acid also functions as a lubricant. The sorbitol content is adjusted to maintain a constant tablet weight. The tablets containing 325 mg. acetylsalicylic acid have the composition of:

| Weight % | Ingredient |
|---|---|
| 10.00 | Acetylsalicylic Acid |
| 27.23 | Citric Acid |
| 14.62 | Sodium Bicarbonate |
| 7.66 | Calcium Carbonate |
| 9.23 | Potassium Bicarbonate |
| 12.31 | Glycine |
| 0.84 | Flavors and Sweeteners |
| 18.07 | Tableting Aids Other Than Lubricants |
| 0.03 | Polyvinyl Pyrrolidone |
| 0.02 | Dimethyl Polysiloxane |
| 0.002 | Dioctyl Sodium Sulfosuccinate |
| 1̅0̅0̅.̅0̅1̅2̅ | |

### EXAMPLE 4

The formulation of Example 3 is modified to increase the tablet content of acetylsalicylic acid to 500 mg. The sorbitol content is reduced to compensate for this keeping all the other ingredients the same. The tablet product has the composition of:

| Weight % | Ingredient |
|---|---|
| 15.00 | Acetylsalicylic Acid |
| 26.56 | Citric Acid |
| 14.25 | Sodium Bicarbonate |
| 7.47 | Calcium Carbonate |
| 9.00 | Potassium Bicarbonate |
| 12.00 | Glycine |
| 0.82 | Flavors and Sweeteners |
| 14.86 | Tableting Aids Other Than Lubricants |
| 0.03 | Polyvinyl Pyrrolidone |
| 0.02 | Dimethyl Polysiloxane |
| 0.002 | Dioctyl Sodium Sulfosuccinate |
| 1̅0̅0̅.̅0̅1̅2̅ | |

### EXAMPLE 5

The formulation of Example 1 is modified to produce a tablet containing 162.5 mg. acetaminophen and 162.5 mg. acetylsalicylic acid. The sorbitol content is adjusted to compensate for this and the fumaric acid is reduced to an amount necessary for adequate lubrication. The tablet product has the composition of:

| Weight % | Ingredient |
|---|---|
| 5.00 | Acetaminophen |
| 5.00 | Acetylsalicyclic Acid |
| 27.43 | Citric Acid |
| 14.62 | Sodium Bicarbonate |
| 7.66 | Calcium Carbonate |
| 9.23 | Potassium Bicarbonate |
| 12.31 | Glycine |
| 0.84 | Flavors and Sweeteners |
| 16.33 | Tableting Aids Other Than Lubricants |
| 1.54 | Fumaric Acid |
| 0.03 | Polyvinyl Pyrrolidone |
| 0.02 | Dimethyl Polysiloxane |
| 0.002 | Dioctyl Sodium Sulfosuccinate |
| 1̅0̅0̅.̅0̅1̅2̅ | |

### EXAMPLE 6

The formulation of Example 5 is modified to increase the tablet content of acetaminophen and acetylsalicylic acid each to 250 mg. The sorbitol content is reduced to compensate for this and the fumaric acid is deleted. The tablet product has the composition of:

| Weight % | Ingredient |
|---|---|
| 7.69 | Acetaminophen |
| 7.69 | Acetylsalicyclic Acid |
| 27.54 | Citric Acid |
| 14.62 | Sodium Bicarbonate |
| 7.66 | Calcium Carbonate |
| 9.23 | Potassium Bicarbonate |
| 12.31 | Glycine |
| 0.84 | Flavors and Sweeteners |
| 12.38 | Tableting Aids Other Than Lubricants |
| 0.03 | Polyvinyl Pyrrolidone |
| 0.02 | Dimethyl Polysiloxane |
| 0.002 | Dioctyl Sodium Sulfosuccinate |
| 1̅0̅0̅.̅0̅1̅2̅ | |

### EXAMPLE 7

The formulation of Example 3 is modified to remove the glycine and the tableting aids. The product has the composition of:

| Weight % | Ingredient |
|---|---|
| 14.36 | Acetylsalicylic Acid |
| 39.11 | Citric Acid |
| 20.99 | Sodium Bicarbonate |
| 11.00 | Calcium Carbonate |
| 13.26 | Potassium Bicarbonate |
| 1.20 | Flavors and Sweeteners |
| 0.04 | Polyvinyl Pyrrolidone |
| 0.02 | Dimethyl Polysiloxane |
| 0.002 | Dioctyl Sodium Sulfosuccinate |
| 9̅9̅.̅9̅8̅2̅ | |

### EXAMPLE 8

The formulation of Example 3 is modified to remove the glycine but retain tableting aids. The overall tablet weight is the same. The product has the composition of:

| Weight % | Ingredient |
|---|---|
| 10.0 | Acetylsalicylic Acid |
| 27.23 | Citric Acid |
| 14.62 | Sodium Bicarbonate |
| 7.66 | Calcium Carbonate |
| 9.23 | Potassium Bicarbonate |
| 0.84 | Flavors and Sweeteners |
| 30.38 | Tableting Aids Other Than Lubricants |
| 0.03 | Polyvinyl Pyrrolidone |
| 0.02 | Dimethyl Polysiloxane |
| 0.002 | Dioctyl Sodium Sulfosuccinate |
| 1̅0̅0̅.̅0̅1̅2̅ | |

The following example describes production of tablets of another form of the preferred composition.

### EXAMPLE 9

A 102 kg. quantity of granulated acetaminophen (containing 95.6 weight percent acetaminophen, 3.8 weight percent citric acid, and 0.6 weight percent hydroxypropylmethyl cellulose) was passed through a Fitzpatrick Comminutor Model D at 4500 rpm. A 64.67 kg. quantity of glycine was dried at 130° F. (54.44° C.) for 16 hr. Potassium bicarbonate granules were prepared by mixing 90 kg. of potassium bicarbonate with 9.9 kg. of 40 weight percent aqueous sodium citrate solution in a Littleford-Lodige Mixer and then drying the resulting granules at 180° F. (82.22° C.) for at least 22 hr. Such granules were then passed through a Fluid Aire Mill operating at 1500 rpm. A premix of 0.3 kg. polyvinyl pyrrolidone, 0.15 kg. dimethyl polysiloxane and 0.015 kg. of dioctyl sodium sulfosuccinate (in the form of a mixture containing 85 weight percent dioctyl sodium sulfosuccinate and 15 weight percent sodium benzoate) was prepared by passing such materials through a Fitzpatrick Comminutor Model D at 4700 rpm. A 40.5 kg. quantity of fumaric acid was passed through a Fitzpatrick Comminutor Model D at 2500 rpm. All of the above materials along with 150 kg. sorbitol, 5.01 kg. of a mixture of lemon, grapefruit and orange flavors, 0.9 kg. calcium saccharin, 11.007 kg. aspartame granules (containing 20.44 weight percent aspartame as described in Example 1), 101.1 kg. spray-dried calcium carbonate (containing 83 weight percent calcium carbonate as described in Example 1), 266.1 kg. anhydrous citric acid, and 139.5 kg. sodium bicarbonate that had been heat-treated as described in U.S. Patent No. 3,105,792 were mixed in an Englesmann Mixer at 20 rpm for 14 minutes. The final mixture was then fed to a tablet press to produce tablets each containing 325 mg. acetaminophen and having a composition of:

| Weight % | Ingredient |
|---|---|
| 10.00 | Acetaminophen |
| 27.69 | Citric Acid |
| 14.31 | Sodium Bicarbonate |
| 8.62 | Calcium Carbonate |
| 9.23 | Potassium Bicarbonate |
| 6.63 | Glycine |
| 0.84 | Flavors and Sweeteners |
| 18.48 | Sorbitol and Other Tableting Aids |
| 4.15 | Fumaric Acid |
| 0.03 | Polyvinyl Pyrrolidone |
| 0.02 | Dimethyl Polysiloxane |
| 0.002 | Dioctyl Sodium Sulfosuccinate |
| 1̅0̅0̅.̅0̅0̅2̅ | |

When the above tablet product was placed in water, there was significant effervescence while the tablet dissolved resulting in a substantially clear solution with no scum on the liquid surface. This solution had a pleasant taste with no undesirable after-taste.

### EXAMPLE 10

The formulation of Example 9 is modified to remove the glycine but retain tableting aids. The overall dose weight is the same. The product has the composition of:

| Weight % | Ingredient |
|---|---|
| 10.00 | Acetaminophen |
| 27.69 | Citric Acid |
| 14.31 | Sodium Bicarbonate |
| 8.62 | Calcium Carbonate |
| 9.23 | Potassium Bicarbonate |
| 0.84 | Flavors and Sweeteners |
| 25.11 | Tableting Aids |
| 4.15 | Fumaric Acid |
| 0.03 | Polyvinyl Pyrrolidone |
| 0.02 | Dimethyl Polysiloxane |
| 0.002 | Dioctyl Sodium Sulfosuccinate |
| 1̅0̅0̅.̅0̅0̅2̅ | |

### EXAMPLE 11

The formulation of Example 9 was modified to increase the tablet content of acetaminophen to 500 mg. The sweetener content was increased and the glycine and tableting aids other than lubricants were adjusted appropriately. The other ingredients remained the same. The tablet product had the composition of:

| Weight % | Ingredient |
|---|---|
| 14.75 | Acetaminophen |
| 26.70 | Citric Acid |
| 13.72 | Sodium Bicarbonate |
| 8.26 | Calcium Carbonate |
| 8.85 | Potassium Bicarbonate |
| 3.93 | Glycine |
| 1.49 | Flavors and Sweeteners |
| 18.41 | Sorbitol and Other Tableting Aids |
| 3.84 | Fumaric Acid |
| 0.03 | Polyvinyl Pyrrolidone |
| 0.01 | Dimethyl Polysiloxane |
| 0.001 | Dioctyl Sodium Sulfosuccinate |
| 9̅9̅.̅9̅9̅1̅ | |

### EXAMPLE 12

The formulation of Example 1 is modified to substitute 6.25 mg. ketoprofen for 325 mg. acetaminophen. The other ingredients remain the same. The tablet product has the composition of:

| Weight % | Ingredient |
|---|---|
| 0.21 | Ketoprofen |
| 30.35 | Citric Acid |
| 16.29 | Sodium Bicarbonate |
| 8.54 | Calcium Carbonate |
| 10.29 | Potassium Bicarbonate |
| 13.72 | Glycine |
| 0.93 | Flavors and Sweeteners |
| 16.36 | Sorbitol and Other Tableting Aids |
| 3.26 | Fumaric Acid |
| 0.03 | Polyvnyl Pyrrolidone |
| 0.02 | Dimethyl Polysiloxane |
| 0.002 | Dioctyl Sodium Sulfosuccinate |
| 1̅0̅0̅.̅0̅0̅2̅ | |

### EXAMPLE 13

The formulation of Example 12 is modified to increase the ketotprofen content to 50 mg. The other ingredients remain the same.

## Claims (Claims for the following Contracting State(s): AT, BE, CH, DE, DK, FR, GB, GR, IT, LI, LU, NL, SE)

1. An effervescent analgesic antacid composition having a reduced sodium content which is capable of being completely dissolved in water to form a pleasant tasting solution which comprises a mixture of 0.2 - 16 % of an analgesic selected from the class consisting of acetylsalicylic acid, acetaminophen, ketoprofen and mixtures thereof, 26 - 40% citric acid, 0 - 14 % glycine, 0.8 - 1.5% flavors and sweeteners, 0 - 31% tableting aids other than lubricants and 0 - 6% tablet lubricant other than acetylsalicylic acid and an antacid component characterized by containing 13 - 21% sodium bicarbonate, 7 - 12 % calcium carbonate and 8 - 14 % potassium bicarbonate, said percents being weight percent based on the total weight of the composition.

2. The composition of Claim 1 suitable for forming tablets which are capable of being dissolved in water to form a pleasant tasting solution which contains 11-31% tableting aids other than lubricants and 1-6% tablet lubricant other than acetylsalicylic acid.

3. The composition of Claim 2 which also contains 0.03-0.04% polyvinyl pyrrolidone, 0.01-0.02% organopolysiloxane and 0.001-0.002% dioctyl sodium sulfosuccinate.

4. The composition of Claim 2 wherein the major component of the tableting aids is sorbitol and the tablet lubricant is fumaric acid.

5. An effervescent analgesic antacid composition according to Claim 1 having a reduced sodium content suitable for forming tablets which are capable of being completely dissolved in water forming a pleasant tasting solution which consists essentially of a mixture of 0.2-16% of an analgesic selected from the class consisting of acetylsalicylic acid, acetaminophen, ketoprofen and mixtures thereof, 26-30% citric acid, 13-16% sodium bicarbonate, 7-9% calcium carbonate, 8-10% potassium bicarbonate, 0-13% glycine, 0.8-1.5% flavors and sweeteners, 11-26% tableting aids other than lubricants, 2-6% fumaric acid, 0.03-0.04% polyvinyl pyrrolidone, 0.01-0.02% organopolysiloxane, and 0.001-0.002% dioctyl sodium sulfosuccinate, said percents being weight percent based on the total weight of the composition.

6. A composition according to Claims 1 or 5 wherein the analgesic is acetaminophen, acetylsalicylic acid or a mixture thereof.

7. A composition according to Claims 1 or 5 wherein the analgesic is ketoprofen.

8. A process for the manufacture of an effervescent analgesic antacid composition having a reduced sodium content which is capable of being dissolved in water to form a pleasant tasting solution which comprises blending 0.2-16% of an analgesic selected from the class consisting of acetylsalicylic acid, acetaminophen, ketoprofen and mixtures thereof, 26-40% citric acid, 13-21% sodium bicarbonate, 7-12% calcium carbonate, 8-14% potassium bicarbonate, 0-14% glycine, 0.8-1.5% flavors and sweeteners, 0-31% tableting aids other than lubricants, and 0-6% tablet lubricant other than acetylsalicylic acid, said percents being weight percent based on the total weight. of the composition.

9. A process for the manufacture of an effervescent analgesic antacid composition according to Claim 8 having a reduced sodium content suitable for forming tablets which are capable of being substantially completely dissolved in water forming a pleasant tasting solution which consists essentially of blending 0.2-16% of an analgesic selected from the class consisting of acetylsalicylic acid, acetaminophen, ketoprofen and mixtures thereof, 26-30% citric acid, 13-16% sodium bicarbonate, 7-9% calcium carbonate, 8-10% potassium bicarbonate, 0-13% glycine, 0.8-1.5% flavors and sweeteners, 11-26% tableting acids other than lubricants, 2-6% fumaric acid, 0.03-0.04% polyvinyl pyrrolidone, 0.01-0.02% organopolysiloxane, and 0.001-0.002% dioctyl sodium sulfosuccinate, said percents being weight percent based on the total weight of the composition.

## Claims (Claims for the following Contracting State(s): ES)

1. A process for the manufacture of an effervescent analgesic antacid composition having a reduced sodium content which is capable of being completely dissolved in water to form a pleasant tasting solution which comprises blending 0.2-16% of an analgesic selected from the class consisting of acetylsalicylic acid, acetaminophen, ketoprofen and mixtures thereof, 26 - 40% citric acid, 13 - 21% sodium bicarbonate, 7 - 12 % calcium carbonate, 8 - 14% potassium bicarbonate, 0 - 14% glycine, 0.8 - 1.5% flavors and sweeteners, 0 - 31% tableting aids other than lubricants, and 0 - 6 % tablet lubricant other than acetylsalicylic acid, said percents being weight percent based on the total weight of the composition.

2. A process for the manufacture of an effervescent analgesic antacid composition according to claim 1 having a reduced sodium content suitable for forming tablets which are capable of being substantially completely dissolved in water forming a pleasant tasting solution which consists essentially of blending 0.2 - 16% of an analgesic selected from the class consisting of acetylsalicylic acid, acetaminophen, ketoprofen and mixtures thereof, 26 - 30% citric acid, 13 - 16% sodium bicarbonate, 7 - 9% calcium carbonate, 8 - 10% potassium bicarbonate, 0 - 13% glycine, 0.8 - 1.5% flavors and sweeteners, 11 - 26% tableting aids other than lubricants, 2 - 6% fumaric acid, 0.03 - 0.04% polyvinyl pyrrolidone, 0.01 - 0.02% organopolysiloxane, and 0.001 - 0.002% dioctyl sodium sulfosuccinate, said percents being weight percent based on the total weight of the composition.

3. A process for the manufacture of an effervescent analgesic antacid composition according to claims 1 or 2 wherein the analgesic is acetaminophen, acetylsalicylic acid or a mixture thereof or is ketoprofen.

## Patentansprüche (Patentansprüche für folgende(n) Vertragsstaat(en): AT, BE, CH, DE, DK, FR, GB, GR, IT, LI, LU, NL, SE)

1. Analgetische antacide Brausezusammensetzung mit reduziertem Natriumgehalt, welche in Wasser vollständig auflösbar ist, wobei eine angenehm schmeckende Lösung gebildet wird, welche eine Mischung aus 0,2-16% Analgetikum, ausgewählt aus der aus Acetylsalicylsäure, Acetaminophen, Ketoprofen und Gemischen der genannten bestehenden Gruppe, 26-40% Citronensäure, 0-14% Glycin, 0,8-1,5% Aroma- und Süßstoffe, 0-31% Tablettierhilfsstoffe, die keine Gleitmittel sind, und 0-6% andere Tablettengleitmittel als Acetylsalicylsäure sowie eine antacide Komponente umfaßt, dadurch gekennzeichnet, daß sie 13-21% Natriumbikarbonat, 7-12% Calciumkarbonat und 8-14% Kaliumbikarbonat enthält, wobei die Prozentangaben Gewichtsprozente sind, die sich auf das Gesamtgewicht der Zusammensetzung beziehen.

2. Zusammensetzung nach Anspruch 1, die zur Herstellung von Tabletten geeignet ist, welche in Wasser löslich sind und dabei eine Lösung mit angenehmem Geschmack bilden, die 11-31% Tablettierhilfsstoffe, die keine Gleitmittel sind, und 1-6% andere Tablettengleitmittel als Acetylsalicylsäure enthält.

3. Zusammensetzung nach Anspruch 2, welche auch 0,03-0,04% Polyvinylpyrrolidon, 0,01-0,02% Organopolysiloxan und 0,001-0,002% Dioktyl-Natriumsulfosuccinat enthält.

4. Zusammensetzung nach Anspruch 2, bei der die Hauptkomponente der Tablettierhilfsmittel Sorbitol und das Tablettengleitmittel Fumarsäure ist.

5. Analgetische antacide Brausezusammensetzung nach Anspruch 1, mit reduziertem Natriumgehalt, die zur Herstellung von Tabletten geeignet ist, welche in Wasser vollständig auflösbar sind und dabei eine Lösung mit angenehmem Geschmack bilden, die im wesentlichen aus einem Gemisch aus 0,2-16% Analgetikum, ausgewählt aus der aus Acetylsalicylsäure, Acetaminophen, Ketoprofen und Gemischen der genannten bestehenden Klasse, 26-30% Citronensäure, 13-16% Natriumbikarbonat, 7-9% Calciumkarbonat, 8-10% Kaliumbikarbonat, 0-13% Glycin, 0,8-1,5% Aroma- und Süßstoffe, 11-26% Tablettierhilfsstoffe, die keine Gleitmittel sind, 2-6% Fumarsäure, 0,03-0,04% Polyvinylpyrrolidon, 0,01-0,02% Organopolysiloxan und 0,001-0,002% Dioctylnatriumsulfosuccinat besteht, wobei die Prozente Gewichtsprozente sind, die sich auf das Gesamtgewicht der Zusammensetzung beziehen.

6. Zusammensetzung nach Anspruch 1 oder 5, bei der das Analgetikum Acetaminophen, Acetylsalicylsäure oder eine Mischung der genannten ist.

7. Zusammensetzung nach Anspruch 1 oder 5, bei der das Analgetikum Ketoprofen ist.

8. Verfahren zur Herstellung einer analgetischen antaciden Brausezusammensetzung mit reduziertem Natriumgehalt, die in Wasser unter Bildung einer angenehm schmeckenden Lösung löslich ist, welches das Vermischen von 0,2-16% Analgetikum, ausgewählt aus der aus Acetylsalicylsäure, Acetaminophen, Ketoprofen und Mischungen der genannten bestehenden Klasse, 26-40% Citronensäure, 13-21% Natriumbikarbonat, 7-12% Calciumkarbonat, 8-14% Kaliumbikarbonat, 0-14% Glycin, 0,8-1,5% Aroma- und Süßstoffe, 0-31% Tablettierhilfsstoffe, die keine Gleitmittel sind, und 0,6% andere Tablettengleitmittel als Acetylsalicylsäure umfaßt, wobei die Prozente Gewichtsprozente sind, die sich auf das Gesamtgewicht der Zusammensetzung beziehen.

9. Verfahren zur Herstellung einer analgetischen antaciden Brausezusammensetzung gemäß Anspruch 8, die einen reduzierten Natriumgehalt hat und die zur Herstellung von Tabletten geeignet ist, welche in Wasser im wesentlichen vollständig unter Bildung einer angenehm schmeckenden Lösung löslich sind, welches im wesentlichen aus dem Vermischen von 0,2-16% Analgetikum, ausgewählt aus der aus Acetylsalicylsäure, Acetaminophen, Ketoprofen und Gemischen der genannten bestehenden Klasse, 26-30% Citronensäure, 13-16% Natriumbikarbonat, 7-9% Calciumkarbonat, 8-10% Kaliumbikarbonat, 0-13% Glycin, 0,8-1,5% Aroma- und Süßstoffe, 11-26% Tablettierhilfsstoffe, die keine Gleitmittel sind, 2-6% Fumarsäure, 0,03-0,04% Polyvinylpyrrolidon, 0,01-0,02% Organopolysiloxan und 0,001-0,002 Gew.% Dioktylnatriumsulfosuccinat besteht, wobei die Prozente Gewichtsprozente sind und sich auf das Gesamtgewicht der Zusammensetzung beziehen.

## Patentansprüche (Patentansprüche für folgende(n) Vertragsstaat(en): ES)

1. Verfahren zur Herstellung einer analgetischen antaciden Brausezusammensetzung mit reduziertem Natriumgehalt, die in Wasser unter Bildung einer angenehm schmeckenden Lösung vollständig löslich ist, welches das Vermischen von 0,2-16% Analgetikum, ausgewählt aus der aus Acetylsalicylsäure, Acetaminophen, Ketoprofen und Mischungen der genannten bestehenden Klasse, 26-40% Citronensäure, 13-21% Natriumbikarbonat, 7-12% Calciumkarbonat und 8-14% Kaliumbikarbonat, 0-14% Glycin, 0,8-1,5% Aroma- und Süßstoffe, 0-31% Tablettierhilfsstoffe, die keine Gleitmittel sind, und 0-6% andere Gleitmittel als Acetylsalicylsäure umfaßt, wobei die Prozente Gewichtsprozente sind, die sich auf das Gesamtgewicht der Zusammensetzung beziehen.

2. Verfahren zur Herstellung einer analgetischen antaciden Brausezusammensetzung nach Anspruch 1, wobei die Zusammensetzung einen reduzierten Natriumgehalt hat und zur Herstellung von Tabletten geeignet ist, welche in Wasser im wesentlichen vollständig unter Bildung einer angenehm schmeckenden Lösung löslich sind, welches im wesentlichen das Vermischen von 0,2-16% Analgetikum, ausgewählt aus der aus Acetylsalicylsäure, Acetaminophen, Ketoprofen und Gemischen der genannten bestehenden Klasse, 26-30% Citronensäure, 13-16% Natriumbikarbonat, 7-9% Calciumkarbonat, 8-10% Kaliumbikarbonat, 0-13% Glycin, 0,8-1,5% Aroma- und Süßstoffe, 11-26% Tablettierhilfsstoffe, die keine Gleitmittel sind, 2-6% Fumarsäure, 0,03-0,04% Polyvinylpyrrolidon, 0,01-0,02% Organopolysiloxan und 0,001-0,002% Dioktylnatriumsulfosuccinat umfaßt, wobei die Prozente Gewichtsprozente sind, die sich auf das Gesamtgewicht der Zusammensetzung beziehen.

3. Verfahren zur Herstellung einer analgetischen antaciden Zusammensetzung nach Anspruch 1 oder 2, wobei das Analgetikum Acetaminophen, Acetylsalicylsäure oder eine Mischung der genannten oder Ketoprofen ist.

## Revendications (Revendications pour l'(les) Etat(s) contractant(s) suivant(s): AT, BE, CH, DE, DK, FR, GB, GR, IT, LI, LU, NL, SE)

1. Composition effervescente analgésique antacide à teneur en sodium réduite, qui est capable d'être entièrement dissoute dans l'eau pour former une solution de goût agréable, qui comprend un mélange de 0,2 à 16 % d'un analgésique choisi dans la classe comprenant l'acide acétylsalicylique, l'acétaminophène, le cétoprofène et des mélanges de ces composés, 26 à 40 % d'acide citrique, 0 à 14 % de glycine, 0,8 à 1,5 % d'arômes et d'édulcorants, 0 à 31 % de substances auxiliaires autres que des lubrifiants pour la formation de comprimés et 0 à 6 % de lubrifiant pour comprimés autre que l'acide acétylsalicylique et un composant antacide caractérisé en ce qu'il contient 13 à 21 % de bicarbonate de sodium, 7 à 12 % de carbonate de calcium et 8 à 14 % de bicarbonate de potassium, ces pourcentages étant exprimés en poids sur la base du poids total de la composition.

2. Composition suivant la revendication 1, convenant pour la formation de comprimés qui sont capables d'être dissous dans l'eau pour former une solution de goût agréable qui contient 11 à 31 % de substances auxiliaires autres que des lubrifiants pour la formation de comprimés et 1 à 6 % de lubrifiant pour comprimés autre que l'acide acétylsalicylique.

3. Composition suivant la revendication 2, qui contient aussi 0,03 à 0,04 % de polyvinylpyrrolidone, 0,01 à 0,02 % d'organopolysiloxane et 0,001 à 0,002 % de dioctylsulfosuccinate de sodium.

4. Composition suivant la revendication 2, dans laquelle le composant principal des substances auxiliaires pour la formation de comprimés est le sorbitol et le lubrifiant pour comprimés est l'acide fumarique.

5. Composition effervescente analgésique antacide suivant la revendication 1, de teneur en sodium réduite, convenant pour la formation de comprimés qui sont capables d'être entièrement dissous dans l'eau en formant une solution de goût agréable, qui est essentiellement constituée d'un mélange de 0,2 à 16 % d'un analgésique choisi dans la classe comprenant l'acide acétylsalicylique, l'acétaminophène, le cétoprofène et des mélanges de ces substances, 26 à 30 % d'acide citrique, 13 à 16 % de bicarbonate de sodium, 7 à 9 % de carbonate de calcium, 8 à 10 % de bicarbonate de potassium, 0 à 13 % de glycine, 0,8 à 1,5 % d'arômes et d'édulcorants, 11 à 26 % de substances auxiliaires de formation de comprimés autres que des lubrifiants, 2 à 6 % d'acide fumarique, 0,03 à 0,04 % de polyvinylpyrrolidone, 0,01 à 0,02 % d'organopolysiloxane et 0,001 à 0,002 % de dioctylsulfosuccinate de sodium, lesdits pourcentages étant exprimés en poids sur la base du poids total de la composition.

6. Composition suivant la revendication 1 ou 5, dans laquelle l'analgésique est l'acétaminophène, l'acide acétylsalicylique ou un mélange de ces substances.

7. Composition suivant la revendication 1 ou 5, dans laquelle l'analgésique est le cétoprofène.

8. Procédé de préparation d'une composition effervescente analgésique antacide de teneur en sodium réduite, qui est capable d'être dissoute dans l'eau en formant une solution de goût agréable, qui consiste à mélanger 0,2 à 16 % d'un analgésique choisi dans la classe comprenant l'acide acétylsalicylique, l'acétaminophène, le cétoprofène et des mélanges de ces substances, 26 à 40 % d'acide citrique, 13 à 21 % de bicarbonate de sodium, 7 à 12 % de carbonate de calcium, 8 à 14 % de bicarbonate de potassium, 0 à 14 % de glycine, 0,8 à 1,5 % d'arômes et d'édulcorants, 0 à 31 % de substances auxiliaires de formation de comprimés autres que des lubrifiants et 0 à 6 % de lubrifiant pour comprimés autre que l'acide acétylsalicylique, lesdits pourcentages étant exprimés en poids par rapport au poids total de la composition.

9. Procédé de préparation d'une composition effervescente analgésique antacide suivant la revendication 8, de teneur en sodium réduite, convenant pour la formation de comprimés qui sont capables d'être dissous pratiquement en totalité dans l'eau pour former une solution de goût agréable, qui consiste essentiellement à mélanger 0,2 à 16 % d'un analgésique choisi dans la classe comprenant l'acide acétylsalicylique, l'acétaminophène, le cétoprofène et des mélanges de ces substances, 26 à 30 % d'acide citrique, 13 à 16 % de bicarbonate de sodium, 7 à 9 % de carbonate de calcium, 8 à 10 % de bicarbonate de potassium, 0 à 13 % de glycine, 0,8 à 1,5 % d'arômes et d'édulcorants, 11 à 26 % de substances auxiliaires de formation de comprimés autres que les lubrifiants, 2 à 6 % d'acide fumarique, 0,03 à 0,04 % de polyvinylpyrrolidone, 0,01 à 0,02 % d'organopolysiloxane et 0,001 à 0,002 % de dioctylsulfosuccinate de sodium, lesdits pourcentages étant exprimés en poids sur la base du poids total de la composition.

## Revendications (Revendications pour l'(les) Etat(s) contractant(s) suivant(s): ES)

1. Procédé de préparation d'une composition effervescente analgésique antacide de teneur en sodium réduite, qui est capable d'être complètement dissoute dans l'eau pour former une solution de goût agréable, qui consiste à mélanger 0,2 à 16 % d'un analgésique choisi dans la classe comprenant l'acide acétylsalicylique, l'acétaminophène, le cétoprofène et des mélanges de ces substances, 26 à 40 % d'acide citrique, 13 à 21 % de bicarbonate de sodium, 7 à 12 % de carbonate de calcium, 8 à 14 % de bicarbonate de potassium, 0 à 14 % de glycine, 0,8 à 1,5 % d'arômes et d'édulcorants, 0 à 31 % de substances auxiliaires de formation de comprimés autres que des lubrifiants et 0 à 6 % de lubrifiant pour comprimés autre que l'acide acétylsalicylique, lesdits pourcentages étant exprimés sur la base pondérale par rapport au poids total de la composition.

2. Procédé de préparation d'une composition effervescente analgésique antacide suivant la revendication 1, de teneur en sodium réduite convenant pour la formation de comprimés qui sont capables d'être dissous pratiquement en totalité dans l'eau pour former une solution de goût agréable, qui consiste essentiellement à mélanger 0,2 à 16 % d'un analgésique choisi dans la classe comprenant l'acide acétylsalicylique, l'acétaminophène, le cétoprofène et des mélanges de ces substances, 26 à 30 % d'acide citrique, 13 à 16 % de bicarbonate de sodium, 7 à 9 % de carbonate de calcium, 8 à 10 % de bicarbonate de potassium, 0 à 13 % de glycine, 0,8 à 1,5 % d'arômes et d'édulcorants, 11 à 26 % de substances auxiliaires de formation de comprimés autres que des lubrifiants, 2 à 6 % d'acide fumarique, 0,03 à 0,04 % de polyvinylpyrrolidone, 0,01 à 0,02 % d'organopolysiloxane et 0,001 à 0,002 % de dioctylsulfosuccinate de sodium, lesdits pourcentages étant exprimés en poids sur la base du poids total de la composition.

3. Procédé de préparation d'une composition effervescente analgésique antacide suivant la revendication 1 ou 2, dans lequel l'analgésique est l'acétaminophène, l'acide acétylsalicylique ou un mélange des deux, ou est le cétoprofène.
